Europäisches Patentamt

European Patent Office

Office européen des brevets

(11) Veröffentlichungsnummer: **0 394 956**
**A1**

## EUROPÄISCHE PATENTANMELDUNG

(21) Anmeldenummer: 90107749.5

(22) Anmeldetag: 24.04.90

(51) Int. Cl.⁵: **C09J 133/02, A61L 15/58,**
**A61K 47/32, A61L 15/44**

(30) Priorität: 26.04.89 DE 3913734

(43) Veröffentlichungstag der Anmeldung:
**31.10.90 Patentblatt 90/44**

(84) Benannte Vertragsstaaten:
**AT BE CH DE DK ES FR GB IT LI NL SE**

(71) Anmelder: **RÖHM GMBH**
**Kirschenallee**
**D-6100 Darmstadt(DE)**

(72) Erfinder: **Beier, Helmut, Dr.**
**Langgässerweg 36**
**D-6100 Darmstadt(DE)**
Erfinder: **Petereit, Hans-Ulrich**
**Lortzingstrasse 22**
**D-6100 Darmstadt(DE)**
Erfinder: **Bergmann, Günter**
**Otto-Grün-Strasse 1**
**D-6450 Hanau 9(DE)**

(54) **Wasserlöslicher druckempfindlicher Hauthaftkleber, dessen Verwendung und damit ausgerüstete Mittel.**

(57) Als druckempfindlicher Hauthaftkleber für Pflaster, für transdermale Arzneiformen oder zur Fixierung von Verbänden, der dauerelastisch und mit Wasser abwaschbar ist, eignet sich ein Salz eines unvernetzten Copolymers aus einer monoäthylenisch ungesättigten Mono- oder Dicarbonsäure und einem Alkylester der Acryl- oder Methacrylsäure, wobei der Anteil der Carbonsäure ausreichen muß, um das Copolymer in der Salzform wasserlöslich zu machen.

EP 0 394 956 A1

Die Erfindung betrifft einen druckempfindlichen Hauthaftkleber zur Befestigung von flächigen flexiblen Substraten, wie Verbänden, Wund- und Heftpflastern oder transdermalen Arzneimitteln, auf der Haut. Von einem druckempfindlichen Kleber spricht man, wenn dieser als getrocknete Schicht dauerhaft weichelastisch ist und beim Aufdrücken eines festen Substrats daran lösbar festklebt. Die getrocknete Kleberschicht braucht jedoch nicht wasserfrei zu sein. Die gewünschte Klebrigkeit tritt manchmal nur dann auf, wenn die Kleberschicht an der Luft abgetrocknet ist und von selbst nicht weiter eintrocknet, jedoch in einem Feuchtigkeitsgleichgewicht mit der Haut steht.

Die Erfindung betrifft weiterhin eine Lösung des Hauthaftklebers und deren Verwendung zum Auftragen auf flexible flächige Substrate. Schließlich betrifft die Erfindung auch Wund- und Heftpflaster und transdermale Arzneimittel.

Stand der Technik

Ein Fixierungsmittel für medizinische Zwecke, bestehend aus einer alkoholischen Lösung von carboxylgruppenhaltigen Polymerisaten oder Mischpolymerisaten polymerisierbarer Äthylenverbindungen und weichmachenden oder harzartigen Stoffen, sowie gegebenenfalls Farb- und Füllstoffen ist bereits aus der DE-PS 855 615 bekannt.

Ein geeignetes Mischpolymerisat ist z.B. aus 40 % Methacrylsäure und 60 % Methylmethacrylat aufgebaut. Da es allein hart und nicht klebend ist, werden auf 2 Teile des Mischpolymerisats 9 Teile Polyvinylmethyläther und 3 Teile Glycerin als Weichmacher mitverwendet. Die Mischung wird aus alkoholischer Lösung auf die Haut aufgetragen und dient zur Fixierung von Verbänden. Der Polymerisatfilm ist wasserbeständig. Da jedoch das Mischpolymerisat in Form seines Alkalisalzes wasserlöslich ist, läßt es sich mit einer schwach alkalischen Seifenlauge von der Haut abwaschen.

EP-B 35 399 beschreibt ein haftfähiges Polyacrylat zur Anwendung auf der Haut, das einen K-Wert von 90 bis 110 hat und zu 16-62 % aus n-Butylacrylat, zu 34-80 % aus 2-Ethylhexylacrylat und zu 4-10 % aus Acrylsäure aufgebaut ist. Durch den hohen Anteil an höheren Acrylestern ist das Mischpolymerisat weich und klebrig und bedarf nicht des Zusatzes eines Weichmachers. Dieses Klebmittel wird aus organischer Lösung, z.B. in Aceton, zu einem druckempfindlichen Klebfilm eines selbstklebenden Pflasters oder Klebstreifens verarbeitet.

Ein Hauthaftkleber, dessen Haftfestigkeit an der Haut stark vom Feuchtigkeitsgehalt abhängt, ist aus WO 84/03837 bekannt. Er enthält ein Mischpolymerisat eines hydrophoben Monomers aus der Gruppe der Alkylester der Acrylsäure mit 4 bis 14 Kohlenstoffatomen im Alkylrest, mit 0,5-30 % eines hydrophilen Monomers aus der Gruppe Acryl- und/oder Methacrylsäure, Acryl- und/oder Methacrylamide, Itakonsäure und Vinylpyrrolidon sowie 5-30 % eines weiteren hydrophilen Monomers, wie Polyethylenglykolacrylat und -methacrylat.

Chem.Abtr. 99, 128 355 referiert die JP-A 83/103 317, betreffend einen Klebfilm für transdermale Arzneimittel, der als Klebharz ein Mischpolymerisat aus 0,1-15 % des Ammoniumsalzes der Acryl- und/oder Methacrylsäure neben anderen Monomeren enthält. Das Klebharz wird nach Wasserzusatz in Form eines Hydrosols auf eine Trägerfolie aufgebracht. Beim Trocknen entweicht Ammoniak, so daß das saure Mischpolymerisat in der Klebschicht zurückbleibt.

Chem.Abstr. 98, 185 617 (JP-A 83/ 15 911) beschreibt ebenfalls einen Klebfilm für transdermale Arzneimittel. Er enthält als Klebharz ein vernetztes, teilweise neutralisiertes Polymerisat einer ungesättigten Carbonsäure, das Glycerin und Gelatine als Weichmacher enthält. Infolge der Vernetzung ist das Klebharz unlöslich.

Dem gleichen Anwendungszweck dienen gemäß Chem.Abstr.97, 188 313 (JP-A 82/134 415) Mischpolymerisate aus Butylacrylat und Ammoniumacrylat, die mit Polyoxypropylensorbitan weichgemacht und mittels Polyepoxidharzen vernetzt werden.

Aufgabe und Lösung

Der Erfindung liegt die Aufgabe zugrunde, einen druckempfindlichen, dauerelastischen Hauthaftkleber zu schaffen, der ohne Anwendung organischer Lösungsmittel aufgetragen und mit Wasser von der Haut entfernt werden kann.

Der erfindungsgemäße Hauthaftkleber enthält ein Salz eines unvernetzten Copolymers aus einer monoäthylenisch ungesättigten, radikalisch polymerisierbaren Mono- oder Dicarbonsäure und wenigstens einem Alkylester der Acryl-und/oder Methacrylsäure. Das Copolymer ist zu einem solchen Anteil aus der ungesättigten Carbonsäure aufgebaut, daß es in der Salzform in Wasser löslich ist.

Das Copolymer gilt im Sinne der Erfindung als wasserlöslich, wenn es in reinem Wasser bei 20 °C eine wenigstens 1-prozentige, klare und homogene Lösung ergibt, in der das Copolymer molekulardispers verteilt vorliegt.

Die dauerelastischen Eigenschaften, welche die Voraussetzung für die Druckempfindlichkeit sind, werden bei einem Feuchtigkeit enthaltenden Film durch eine geeignete Auswahl der Monomerkomponenten des zugrundeliegenden Copolymers oder durch den Zusatz von organischen Weichmachern oder durch eine Kombination dieser Maßnahmen erreicht. Die bevorzugten Hauthaftkleber der Erfindung enthalten 2 bis 200 Gew.-% eines organischen Weichmachers, bezogen auf das Gewicht des Copolymers. Weiterhin können sie mit Hydrophilierungsmitteln, wie Glycerin, Harnstoff oder Sorbitol, versetzt werden, die nur eine begrenzte eigene Weichmacherwirkung haben, aber die Verdunstung des Wassers aus der Kleberschicht verhindern und dadurch die dauerhafte Weichmachung durch den Feuchtigkeitsgehalt gewährleisten. Diese Mittel können in Mengen von z.B. 10 Gew.-% bis zum Mehrfachen des Copolymergewichts angewendet werden.

Wasser hat auf die erfindungsgemäß eingesetzten Copolymeren eine deutlich weichmachende Wirkung, die bei der Anwendung auf der Haut in der Regel zur Wirkung kommt, da die Hauthaftkleberschicht im Feuchtigkeitsgleichgewicht mit der Haut steht. Bei der Herstellung von Hauthaftkleberschichten auf Substraten ist es aus dem gleichen Grund nicht zweckmäßig, die Schicht über diesen Gleichgewichtswert hinaus zu trocknen. Die erwünschte Klebrigkeit tritt in der Regel bei einem Feuchtigkeitsgehalt von 1 bis 10 Gew.-%, bezogen auf das wasserfreie Copolymer, auf.

Der Hauthaftkleber gilt im Sinne der Erfindung als druckempfindlich klebend, wenn er bei dem Klebtest nach European Pharmacopoeia, Second Edition, Part II-7, S. 273 ff (1984) eine Klebkraft von mindestens 1 N/cm aufweist (peel method).

Ausführung der Erfindung

Es ist bekannt, daß die höheren Alkylester der Acryl-und/oder Methacrylsäure den daraus hergestellten Copolymeren Weichheit und Klebrigkeit verleihen. Gleichzeitig machen sie das Copolymer hydrophob und wasserunlöslich. Bei der Festlegung des Anteils der monoäthylenisch ungesättigten, radikalisch polymerisierbaren Carbonsäure ist daher darauf zu achten, daß er einerseits hoch genug ist, um die Löslichkeit in Wasser zu gewährleisten, andererseits niedrig genug ist, um für einen ausreichenden Anteil des weichmachenden Alkylacrylats oder -methacrylats Raum zu lassen. Wenn die zum Aufbau des Copolymeren verwendeten Monomeren bei keinem Mischungsverhältnis diese beiden Eigenschaften gleichzeitig erreichen lassen, kann ein Weichmacher zuhilfe genommen werden.

Die monoäthylenisch ungesättigte, radikalisch polymerisierbare Mono- oder Dicarbonsäure hat vorzugsweise die Struktur

$$R - CH = CR' - COOH$$

worin entweder
R ein Wasserstoffatom und R' ein Wasserstoffatom, eine Methylgruppe oder eine Gruppe -CH$_2$-

COOH oder
R eine Gruppe -COOH und R' ein Wasserstoffatom bedeuten. Zu diesen Carbonsäuren gehören Acryl- und Methacrylsäure, Itakon-, Malein- und Fumarsäure. Der Anteil der monoäthylenisch ungesättigten Mono- oder Dicarbonsäure liegt vorzugsweise bei 30 bis 80 Gew.-%, insbesondere 50 bis 70 Gew.-% des Mischpolymerisats.

Es ist nicht grundsätzlich erforderlich, daß alle Carbonsäureeinheiten des Mischpolymerisats in der Salzform vorliegen, sondern es genügt ein Anteil, der die Wasserlöslichkeit gewährleistet. Der dafür erforderliche Anteil hängt von der Größe und Hydrophobie des Esteranteils ab. In manchen Fällen reicht ein Gehalt von 15 Gew.-% an Carboxylat-Einheiten schon aus, um das Mischpolymerisat wasserlöslich zu machen. In der Regel liegt der Gehalt an Carboxylat-Monomereinheiten bei 20 bis 50 Gew.-%.

Wasserlöslichkeit des Polymerisats läßt sich durch eine Teilneutralisation der vorhandenen Carboxylgruppen erreichen, die je nach dem Gehalt an Carbonsäure-Monomereineheiten zwischen 20 und 50 Mol-% liegt. Für die praktische Anwendung werden Neutralisationsgrade von 50 bis 100, insbesondere 70 bis 100 Mol-% bevorzugt. Mittels des Neutralisationgrades lassen sich die Viskosität der Kleberlösung und die Klebkraft des Haftkleberfilms beeinflussen.

Als salzbildendes Kation sind Alkalikationen, insbesondere Natrium und Kalium, an erster Stelle zu nennen. Andere Metallkationen sind nur insoweit verwendbar, wie sie nicht durch Mehrwertigkeit vernetzend wirken oder eine unerwünschte physiologische Wirksamkeit haben. Organische Ammoniumkationen sind geeignet, wenn sie nicht beim Trocknen oder bei der Lagerung in Form des entsprechenden Amindampfes entweichen. Brauchbar sind z.B. quartäre Ammoniumkationen, wie Tetramethylammonium, oder Ammoniumkationen, die sich von schwer flüchtigen, physiologisch unbedenklichen Aminen, wie Diethanolamin oder Triethanolamin, ableiten.

Unter den Alkylestern der Acrylsäure und/oder Methacrylsäure sind diejenigen mit 1 bis 12 Kohlenstoffatomen im Alkylrest bevorzugt, vor allem die Ester der Acrylsäure. Insbesondere Methyl-, Ethyl-, n-Butyl-, n-Hexyl-, n-Octyl-, 2-Äthyl-hexyl-, n-Decyl- und n-Dodecylacrylat sind gut geeignet. Die niederen Ester der Acryl- und/oder Methacrylsäure werden in der Regel nur als Comonomere neben den höheren Estern eingesetzt. Daneben können weitere Comonomere am Aufbau des Copolymers beteiligt sein, sofern sie die Wasserlöslichkeit nicht in unzulässiger Weise herabsetzen oder die Härte unzulässig erhöhen. Beispiele sind Acryl- und/oder Methacrylamid, Hydroxyalkylester und Polyalkylenglykolester der Acryl-und/oder Methacrylsäure, Äthylen, Vinyacetat, Vinylpropionat und Vinylpyrrolidon. Zur Herstellung brauchbarer Hauthaftkleber ist die Mitverwendung dieser Monomerer in der Regel nicht erforderlich; ihr Anteil liegt üblicherweise unter 20 Gew.-%.

Das Copolymer kann in der Salzform durch radikalische Copolymerisation der neutralisierten Carbonsäure mit den anderen Monomerbestandteilen in wäßriger Lösung erzeugt werden. Es wird jedoch bevorzugt, zunächst das nicht neutralisierte Copolymer herzustellen, indem man die freie Mono- oder Dicarbonsäure einsetzt. Für die radikalische Polymerisation dieser Monomerengemische stehen verschiedene, seit langem bekannte Polymerisationsverfahren zur Verfügung, z.B. die Polymerisation in Wasser oder in organischen Lösungsmitteln, die Polymerisation in Substanz und, da die Copolymeren in der Säureform weniger wasserlöslich sind, auch die Emulsionspolymerisation in wäßriger Phase. Die organischen Polymerisatlösungen und wäßrigen Polymerisatdispersionen lassen sich z.B. durch Sprühtrocknung in Pulverprodukte überführen. Die Substanzpolymerisate werden im Extruder aufgeschmolzen und zu einem feinen Granulat extrudiert.

Das Molekulargewicht des Copolymers beeinflußt die Viskosität der wäßrigen Lösung des Hauthaftklebers in Abhängigkeit von der Konzentration, bezogen auf den flüssigen Bestandteil. Es liegt vorzugsweise im Bereich zwischen 20.000 und 2,5 Millionen. Die Viskosität der wäßrigen Lösung des Copolymeren soll nicht mehr als 100 Pa s, vorzugsweise 10 mPa s bis 10 Pa s betragen, wobei der Polymerisatgehalt der Lösung zweckmäßig etwa 20 bis 70 Gew.-% ausmacht.

Die wäßrige Lösung kann dadurch hergestellt werden, daß man das feinteilige Copolymer in der Säureform in einer wäßrige Lösung einer Base rührt, wobei es sich unter Salzbildung auflöst. Der fertigen Lösung können gegebenenfalls Weichmacher und andere Zusätze zugemischt werden. Als Weichmacher eignen sich flüssige, wenigstens begrenzt wasserlösliche organische Stoffe, die mit dem Polymerisat verträglich und unter den Verarbeitungs- und Anwendungsbedingungen nicht oder nicht wesentlich flüchtig sind. Weiterhin sollen sie nicht toxisch sein und nicht bevorzugt in die Haut oder das aufgeklebte Substrat einwandern. Eine ausreichende Verträglichkeit ist gegeben, wenn sich aus dem Copolymeren und dem Weichmacher eine homogene Lösung und aus dieser durch Trocknen ein klarer Film herstellen lassen oder wenn sich das Copolymer in einem Überschuß des Weichmachers lösen läßt. Als Weichmacher geeignete Stoffe haben in der Regel ein Molekulargewicht zwischen 200 und 20.000 und enthalten eine oder mehrere hydrophile Gruppen im Molekül, z.B. Hydroxy-, Äther- oder Aminogruppen.

Beispiele geeigneter Weichmacher sind Citronensäure-triäthylester oder -tributylester, Glycerintriacetat und Polyäthylenglykole 200 bis 20.000.

Anwendung der wäßrigen Hauthaftkleberlösung

Aufgrund ihrer nicht-toxischen und nicht reizenden Eigenschaften kann die wäßrige Hauthaftkleberlösung zur Bildung einer klebenden Oberfläche direkt auf die Haut aufgetragen werden. Nach kurzer Trocknung kann die Klebschicht zur Fixierung von Verbänden, Stützstrümpfen, Korsagen etc. verwendet werden, indem sie auf die Schicht leicht aufgedrückt werden. Das aufgeklebte Substrat kann nach Abschluß der vorgesehenen Einwirkungszeit einfach abgezogen werden, was sich gegebenenfalls durch Anfeuchten mit Wasser erleichtern läßt. Auch bei häufig wiederholten Beschichtungen der gleichen Hautstellen wird keine Reizung beobachtet. Infolge des hydrophilen Charakters der Klebschicht wird die Haut deutlich weniger in Mitleidenschaft gezogen als beim Einsatz konventioneller Kautschukklebschichten. Die auf der Haut verbliebene Klebschicht einschließlich anhaftendem Schutz, Gewebefasern usw. läßt sich leicht und rasch mit kaltem oder warmem Wasser abwaschen.

Die wäßrige Hauthaftkleberlösung eignet sich in gleicher Weise zur Erzeugung von hauthaften druckempfindlichen Klebschichten von Wundpflastern, medizinischen Heftpflastern und Klebebändern oder von transdermal wirkenden, zum Aufbringen auf die Haut vorgesehenen Arzneimitteln. Sie können einen topischen oder systemischen pharmazeutischen Wirkstoff enthalten, der in der Kleberschicht selbst oder in dem beschichteten Substrat enthalten sein kann. Bis zur Anwendung wird die getrocknete Klebschicht, die noch eine ausreichende Feuchtigkeitsmenge enthält, zweckmäßig mit einer Trennfolie geschützt.

Um die Klebschicht zu erzeugen, wird eine 0,01 bis 1 mm dicke Schicht der wäßrigen Hauthaftkleberlösung auf das Substrat aufgebracht und beispielsweise 1 min bis 24 Stunden bei 20 bis 100 °C getrocknet, wobei der zur Aufrechterhaltung der gewünschten Klebrigkeit erforderliche Feuchtigkeitsgehalt nicht unterschritten werden soll. Gewünschtenfalls kann die Beschichtung mehrmals wiederholt werden, um eine größere Dicke zu erreichen. Zur Beschichtung kommen flexible flächige Substrate in Betracht, z.B. die bekannten, für Heftpflaster gebräuchlichen Gewebe, Vliese oder Folien aus Kunststoff oder Metall, besonders Aluminium. Ebenso können spezielle Polymerschichten, die z.B. als Wirkstoffreservoir dienen, sowie deren Laminate mit Metallfolien mit einer Klebschicht versehen werden. Beim technischen Aufbringen der erfindungsgemäßen Hauthaftkleber wird es als vorteilhaft empfunden, daß keine umweltbelastenden oder explosiven Lösungsmitteldämpfe aus der Trockenzone zu entsorgen sind und daß die Beschichtungsanlage allein mit Wasser gereinigt werden kann.

BEISPIELE

Beispiel 1

12,5 g Natriumhydroxid werden in 265,5 g gereinigtem Wasser gelöst. Zu diesem Ansatz gibt man 70,5 g eines anionischen Copolymerisates aus Ethylacrylat und Methacrylsäure (Gewichtsverhältnis 1 : 1, Handelsprodukt ®EUDRAGIT L 100-155, Röhm GmbH) und löst es unter ständigem Rühren auf. Abschließend werden 106,0 g Polyethylenglykol (Mol.-Gew. 400) und 45,6 g wasserfreies Glycerin zugemischt.
Die Lösung mit einem Trockengehalt von 46,9 Gew.-% hat eine Viskosität von 1925 mPas (nach Brookfield, II/6/20). Sie wird mittels einer Rakel auf eine Aluminium-Folie mit einer Naßschichtdicke von 500 µm aufgetragen und bei 80 Grad C getrocknet. Der Kleberauftrag liegt bei ca. 13 mg/qcm. Die Kleberschicht erreicht eine Klebkraft von 5,4 N/cm.

Beispiel 2

282 g eines anionischen Copolymerisates aus Methylmethacrylat und Methacrylsäure (Gewichtsverhältnis 1 : 1, Handelsprodukt ®EUDRAGIT L 100, Röhm GmbH) werden in 762 g gereinigtem Wasser suspendiert und unter Rühren 10 min. vorgequollen. Mit diesem Ansatz mischt man eine Lösung aus 50 g Natriumhydroxid in 300 g gereinigtem Wasser und rührt bis die Lösung des Polymers klar ist. Anschließend werden 424 g Polyethylenglykol (Mol.Gew. 400) und 182 g wasserfreies Glycerin zugemischt. Die Lösung hat einen Trockengehalt von 46,9 % und eine Viskosität von 1650 mPas (nach Brookfield, /6/20). Jeweils 60 ml dieser Formulierung werden in Roll-on-Stifte abgefüllt.
Trägt man die Lösung auf die Haut auf, so bildet sich nach dem Abtrocknen eine klebrige Schicht, die Stützstrümpfe sicher fixiert. Auch nach mehreren Tagen zeigten sich keine Hautirritationen. Klebereste auf der Haut und dem Strumpfgewebe lassen sich mit Wasser leicht abwaschen.

Beispiel 3

50 g eines anionischen Copolymerisates aus Methacrylsäuremethylester und Methacrylsäure (Gewichtsverhältnis 1 : 2, Handelsprodukt ®EUDISPERT hv, Röhm GmbH) werden in 450 g gereinigtem Wasser suspendiert und unter Rühren 10 min. vorgequollen. Mit diesem Ansatz mischt man eine Lösung von 12 g Natriumhydroxid in 238 g gereinigtem Wasser und rührt, bis die Lösung des Polymers klar ist. Anschließend werden 50 g Triethylcitrat und 200 g wasserfreies Glycerin zugemischt. Dieses Gel trocknet auf der Haut zu einer klebrigen Schicht, die Gewebe oder Verbände gegen Verrutschen fixiert.

Beispiel 4

65,6 g eines anionischen Copolymerisats aus Methylmethacrylat und Methacrylsäure (Gewichtsverhältnis 1 : 1, Handelsprodukt ®EUDRAGIT L 100, Röhm GmbH) werden in 239,2 g gereinigtem Wasser unter Rühren auf 80 bis 90 Grad C erhitzt und mit 54,3 g Triethanolamin versetzt. Der Ansatz wird unter weiterem Rühren auf 30 bis 35 Grad C abgekühlt und mit 98,4 g Polyethylenglykol (Mol.Gew. 400) sowie 42,5 g wasserfreiem Glycerin vermischt. Die Lösung hat einen Trockengehalt von 52,2 Gew.-% und eine Viskosität von 1350 mPas (nach Brookfield, II/6/20). Sie wird mittels einer Rakel auf eine Aluminium-Folie mit einer Naßschichtdicke von 500 µm aufgetragen und bei 80 Grad C getrocknet. Die Kleberschicht erreicht mit einer Auftragsmenge von 13 mg/qcm eine Klebkraft von 2,5 N/cm.

Beispiel 5

67,5 g eines anionischen Copolymerisates aus Ethylacrylat und Methacrylsäure (Gewichtsverhältnis 1 : 1, Handelsprodukt ®EUDRAGIT L 100-55, Röhm GmbH) werden in 100 g gereinigtem Wasser suspendiert und unter Rühren 10 min. vorgequollen. Mit diesem Ansatz mischt man eine Lösung aus 12 g Natriumhydroxid und 60 g gereinigtem Wasser und rührt bis die Lösung des Polymers klar ist. Anschließend werden 101,5 g Polyethylenglykol (Mol.Gew. 400) und eine Lösung, bestehend aus 67,5 g Harnstoff und 91,5 g gereinigtem Wasser, zugemischt. Die Lösung wird mittels einer Rakel auf eine Weich-PVC-Folie (Guttagena-Folie WK 68 SLIM, Fa. Kalle, D-6200 Wiesbaden) mit einer Naßschichtdicke von 500 µm aufgetragen und bei 80 Grad C getrocknet. Die Folie haftet mehrere Stunden auf der menschlichen Haut. Die Kleberschicht mit einer Auftragsmenge von 13 mg/qcm erreicht eine Klebkraft von 5,0 N/cm.

**Ansprüche**

1. Dauerelastischer, druckempfindlicher Hauthaftkleber, enthaltend ein Salz eines unvernetzten Copolymers aus einer monoäthylenisch ungesättigten, radikalisch polymerisierbaren Mono- oder Dicarbonsäure und wenigstens einem Alkylester der Acryl- und/oder Methacrylsäure,
dadurch gekennzeichnet,
daß das Copolymer einen solchen Anteil der ungesättigten Carbonsäure enthält, daß es in der Salzform in Wasser löslich ist.

2. Hauthaftkleber nach Anspruch 1, dadurch gekennzeichnet, daß das Copolymer einen solchen Anteil der ungesättigten Carbonsäure enthält, daß es bei 20- bis 50-prozentiger Neutralisation der Carbonsäureeinheiten in Wasser löslich ist.

3. Hauthaftkleber nach Anspruch 1 oder 2, dadurch gekennzeichnet, daß die monoäthylenisch ungesättigte, radikalisch polymerisierbare Mono- oder Dicarbonsäure die Struktur
$$R - CH = CR' - COOH$$
hat, worin entweder
R ein Wasserstoffatom und $R'$ ein Wasserstoffatom, eine Methylgruppe oder eine Gruppe -CH$_2$-COOH oder
R eine Gruppe -COOH und $R'$ ein Wasserstoffatom bedeuten.

4. Hauthaftkleber nach einem oder mehreren der Ansprüche 1 bis 3, dadurch gekennzeichnet, daß das Copolymer 30 bis 80 Gew.-% der äthylenisch ungesättigten, radikalisch polymerisierbaren Carbonsäure enthält.

5. Hauthaftkleber nach einem oder mehreren der Ansprüche 1 bis 4, dadurch gekennzeichnet, daß das Copolymer einen Alkylester der Acryl- und/oder Methacrylsäure mit 1 bis 14 Kohlenstoffatomen im Alkylrest enthält.

6. Hauthaftkleber nach einem oder mehreren der Ansprüche 1 bis 5, dadurch gekennzeichnet, daß die Carboxylgruppen des Mischpolymerisats zu 50 bis 100 Mol-% neutralisiert sind.

7. Hauthaftkleber nach einem oder mehreren der Ansprüche 1 bis 6, dadurch gekennzeichnet, daß er 2 bis 200 Gew.-% eines Weichmachers für das Salz des Copolymers enthält.

8. Hauthaftkleber nach einem oder mehreren der Ansprüche 1 bis 7, dadurch gekennzeichnet, daß er 1 bis 10 Gew.-% Wasser enthält.

9. Wäßrige Lösung, enthaltend einen Hauthaftkleber nach einem oder mehreren der Ansprüche 1 bis 8 und 20 bis 90 Gew.-% Wasser.

10. Verwendung der wäßrigen Lösung des Hauthaftklebers nach Anspruch 9 zum Auftragen auf eine flächiges flexibles Substrat und anschließende Trocknung.

11. Wundpflaster, enthaltend einen Hauthaftkleber nach einem oder mehreren der Ansprüche 1 bis 8.

12. Transdermal wirkendes, an der Haut haftendes Arzneimittel, enthaltend einen topischen oder systemischen pharmazeutischen Wirkstoff und einen Hauthaftkleber nach einem oder mehreren der Ansprüche 1 bis 8.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | WPI, File supplier, Derwent Publications Ltd., London, GB; AN 73-65267U & JP-B 73-33973 (Nitto Electric Ind Co Ltd) * das ganze Dokument * | 1, 9 | C09J133/02 A61L15/58 A61K47/32 A61L15/44 |
| A | PATENT ABSTRACTS OF JAPAN vol. 11, no. 206 (C-433) 3 Juli 87, & JP-A-62 29516 (NITTO ELECTRIC IND CO LTD) 7 Februar 1987, * das ganze Dokument * | 1, 9 | |

**RECHERCHIERTE SACHGEBIETE (Int. Cl.5 )**

C09J
A61L

Der vorliegende Recherchenbericht wurde für alle Patentansprüche erstellt

| Recherchenort | Abschlußdatum der Recherche | Prüfer |
|---|---|---|
| DEN HAAG | 16 JULI 1990 | SCHUELER D.H.H |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
......................................................................................
& : Mitglied der gleichen Patentfamilie, übereinstimmendes Dokument

EPO FORM 1503 03.82 (P0403)